# EUROPEAN PATENT SPECIFICATION

(11) **EP 3 939 653 B1**
(45) Date of publication and mention of the grant of the patent: **14.01.2026**
(21) Application number: 20185407.2
(22) Date of filing: 13.07.2020
(51) Int. Cl.: A61N 5/06

(54) **GERMICIDAL UV IRRADIATION APPARATUS**
KEIMTÖTENDE UV-BESTRAHLUNGSVORRICHTUNG
APPAREIL D'IRRADIATION GERMICIDE UV

(43) Date of publication of application: 19.01.2022
(73) Proprietor: GME German Medical Engineering GmbH, 91054 Erlangen (DE)
(72) Inventor: FISCHER, Dietmar, Dr., 91056 Erlangen (DE); SCHULZE, Stefan, Dr., 90491 Nürnberg (DE)
(74) Representative: Samson & Partner Patentanwälte mbB

(56) References cited:
- WO-A1-2009/059270
- WO-A1-2012/122210
- WO-A2-03/047682
- US-A1- 2017 290 932

## Description

### FIELD

The present invention generally relates to the field of radiation-based treatment devices and, particularly, to devices using UV-C radiation.

### BACKGROUND

Short-wavelength ultraviolet (UV-C) radiation may be used for disinfection. UV-C radiation has been found to efficiently destroy and/ or inactivate microorganisms by destroying nucleic acids and disrupting their DNA. Because of this, UV-C radiation has been used for purposes of disinfecting objects, food, water or air. Generally, UV-C radiation is defined as UV radiation with a wavelength in a wavelength range between 100 nm and 280 nm. International Patent Application Publication WO 2012/122210 A1 discloses an ultraviolet irradiator which emits ultraviolet radiation to selectively affect bacteria without harming human cells.
International Patent Application Publication WO 2009/059270 A1 discloses a light device which allows to apply germicidal light in order to prevent and treat infections, diseases, and disorders of the body.

### DESCRIPTION

The present invention relates to a germicidal ultraviolet (UV) irradiation apparatus for irradiation of target tissue of a living body. The UV germicidal irradiation apparatus comprises an UV germicidal radiation source, a filter arrangement, a radiation interface, and a radiation output guide. The UV germicidal radiation source is adapted to output UV germicidal radiation in a wavelength range between 190 nm and 230 nm. The UV germicidal radiation source and the filter arrangement are radiation-wisely coupled to transmit radiation from the UV germicidal radiation source to the filter arrangement. The filter arrangement is adapted to remove radiation in a wavelength range between 240 nm and 280 nm from radiation received from the UV germicidal radiation source. The filter arrangement and the radiation interface are radiation-wisely coupled to transmit radiation from the filter arrangement to the radiation interface. The radiation interface being adapted to output radiation received from the filter arrangement. The radiation interface and the radiation output guide are connected (e.g. releasably or not releasably, fixed) to each other and radiation-wisely coupled to transmit radiation from the radiation interface to the radiation output guide. The radiation output guide extends from the radiation interface in a longitudinal direction and is adapted to guide radiation towards target tissue.

The UV germicidal irradiation apparatus may provide irradiation for mainly superficial irradiation with low penetration depth of target tissue. The irradiation apparatus may provide irradiation for irradiating tissues, which enclose fluids such as gases, air or liquids and/or may be at least partly covered or embedded by or immersed in such a fluid.

The UV germicidal irradiation apparatus may be configured to irradiate target tissue of a living body, which can be a human living body, an animal living body or another living creature, such as a plant.

Target issue may comprise external tissue, such as skin, and/or also tissue inside the human body, such as tissue of/in body orifices, body cavities and/ or organs. Target tissue may further comprise fluids enclosed by the respective body orifices or body cavities.

The UV germicidal radiation source of the germicidal radiation apparatus may be adapted to output one of or a combination of single line and narrow spectral emission short-wavelength UV radiation in the range between 190 nm and 230 nm.

The UV germicidal radiation source may further be adapted to output UV germicidal radiation in any sub range of the wavelength range between 190 nm and 230 nm, for example in at least one of a subrange between 190 nm and 210 nm; in a subrange between 210 nm and 230 nm; in a subrange between 215 nm and 225 nm, in a subrange between 221 nm and 223 nm. The radiation source may be adapted to output a radiation of a wavelength in any of the range of 222 nm and the range of 207 nm.

The radiation source may comprise at least one of the following: a UV-C lamp, a UV-C laser, a UV-C LED, an excimer laser, an excimer lamp, a Kr-Br excimer lamp, a Kr-Br excimer laser, a Kr-Cl excimer lamp, and a Kr-Cl excimer laser.

The filter arrangement may be adapted to remove any carcinogenic radiation while transmitting germicidal radiation of the radiation source. The filter arrangement may be adapted to remove radiation in a wavelength range between 240 nm and 280 nm from radiation received from the UV germicidal radiation source. Alternatively, or in combination therewith, the filter arrangement may be adapted to remove radiation of at least one wavelength band in the wavelength range between 240 nm and 280 nm. The filter arrangement may be in particular adapted to remove radiation of a wavelength of 254 nm.

The radiation interface and the radiation output guide may be releasably coupled to each other. The radiation interface and the radiation output guide may be non-releasably coupled to each other. "Releasably connected" preferably indicates a structural, non-permanent connection between the radiation interface and the radiation output guide thereby allowing to combine different radiation output guides with an irradiation apparatus. The non-permanent connection enables a more precise configuration of the irradiation apparatus to specific target tissue. The connection may sufficiently secure the radiation output guide for treatment while allowing simple replacement of the radiation output guide with another radiation output guide. The non-permanent connection may also ensure that the radiation interface and the radiation output guide are radiation-wisely coupled. The non-permanent connection may comprise, for instance, any of a plug-in connection, bolt connection, screw connection, latching connection. The radiation interface may thus enable both, structurally connecting the radiation output guide and the irradiation apparatus, and radiation-wisely coupling of the radiation output guide and the filter arrangement.

The radiation output guide may be extending straight, it may be curved or shaped in another way such that it can be introduced into body orifices.

The filter arrangement may comprise at least one optical filter for optically filtering-out at least one wavelength in at least one predefined wavelength range. The filter arrangement may comprise at least one of a spectrum filter, a multilayer dielectric filter, a chemical filter, a mechanical filter, a dichroic filter, a monochromatic filter or an absorptive filter.

The filter arrangement may comprise at least one collimator adapted to output, in a radiation downstream direction, collimated radiation.

"Radiation downstream direction" "downstream" may be used to indicate the direction of the radiation path from the radiation source through the irradiation apparatus towards target tissue. "Radiation upstream direction" or "upstream" may be used to indicate the opposite direction respectively.

The at least one collimator may be adapted to output, in the radiation downstream direction, collimated radiation essentially in the longitudinal direction. The collimator may be adapted to produce radiation parallel to a main radiation direction. The collimator may remove radiation from the radiation source that exceeds a certain angle with respect to the main radiation direction.

The filter arrangement may comprise at least one optical filter radiationally upstream the at least one collimator. The filter arrangement may comprise at least one collimator radiationally upstream the at least one optical filter.

The filter arrangement may comprise at least one optical filter radiationally upstream the at least one collimator and at least one optical filter radiationally downstream the at least one collimator. The filter arrangement may comprise at least one collimator radiationally upstream the at least one optical filter and at least one collimator radiationally downstream the at least one optical filter. The apparatus may also comprise differing filter arrangements, e.g. with the same number of collimators and optical filters.

The collimator may comprise at least one of non-reflective plastic or ceramic. The collimator may be formed as at least one of a honey-combed structure, a circularly perforated structure, a square-shape perforated structure, and a grid-like structure.

The radiation output guide may comprise at least one the following: a fused silica glass, silica, transparent UV-C material, a metal-coated (hollow) waveguide, waveguide having total-reflection properties, a UV-C transparent sapphire, a UV-C transparent diamond, and a UV-C transparent semi-conductor material.

The radiation output guide may be formed as a solid rod, an optical waveguide, an optical fiber, a tube, a sphere, a hemisphere, a part of a sphere, and a cone.

The radiation output guide may be formed of one or more elements. The one or more elements of the radiation guide may be formed of the same material or different materials. The one or more elements of the radiation output guide may be formed in the same way or in different ways.

The radiation output guide comprises at least one flexible segment. "Flexible" may be used to indicate that the at least one segment is not rigid, but has a shape that can be passively and/or actively altered. For example, the at least one flexible segment may be bendable, extendable, compressible, deformable movable or the like under external force and/or torque applied onto the flexible segment and/or force and/or torque generated by an actuation device of the irradiation apparatus. The radiation output guide may be partially or fully flexible, wherein the at least one flexible part is movable in controlled manner. In the latter respect, the radiation output guide includes an actuating device, which is operatively coupled to the flexible segment of the radiation output guide. The actuating device is adapted to, upon actuation, move the flexible segment of the radiation output guide. The actuating device, as used for/in a flexible endoscope, may comprise a control interface (e.g. button, lever, etc.) for a user to control movements of the output guide, which may be arranged at a housing of the irradiation apparatus.

In some cases, the shape of the flexible segment may be changed during application of UV germicidal irradiation on target tissue. In addition, or as alternative, the shape of the flexible segment may be changed as long as no UV germicidal irradiation is applied.

The flexible segment may be formed so that its present shape is maintained until it changed into a new/different shape, which is then maintained until a further shape change. In such cases, the flexible segment may exhibit a certain firmness to maintain its current shape.

The UV germicidal irradiation apparatus may further comprise at least one tissue parameter determining device being adapted to determine at least one of the following: a temperature of tissue, a color of tissue, a melanin content of tissue, a thickness of tissue, a pathogen burden of tissue, and a germ burden of tissue.

In addition, or alternatively thereto, the irradiation apparatus may further comprise a photodynamic imaging device and/ or an imaging device, which is adapted to least one of the following: generate images of a surface of tissue, generate images of internal structures of tissue, visualize a pathogen burden of tissue, and to visualize a germ burden of tissue.

The imaging device may comprise at least one of an imaging device including a video camera, a microscope, an imaging device using optical coherence tomography, and an imaging device using ultrasound. These imaging techniques may provide imaging data to a display and/ or memory of the UV germicidal irradiation apparatus. Alternatively, or in combination thereto, the imaging techniques may provide imaging data to a display and/ or memory external to the UV germicidal irradiation apparatus.

The UV germicidal irradiation apparatus may further comprise a substance application device being adapted to apply a substance onto tissue. The substance may comprise medical substances that enhance the effect of the germicidal radiation and/ or progress treatment and healing of respective target tissue. In particular, the substance may improve the penetration depth of the germicidal radiation into the tissue, which is also known as optical clearing. The substance may also or alternatively comprise substances that have a physiological or cosmeti-cal effect on the target tissue. The substance may also comprise water.

The irradiation apparatus may comprise a power supply unit and/ or a power supply interface. The power supply unit may comprise batteries, which can be rechargeable. The power supply interface may comprise a charging circuit for wire or wireless charging of the battery. The irradiation apparatus may comprise a control panel with an indicator indicating the charging status of the battery. Alternatively, or in combination thereto, the power supply interface is to be connected to an external power supply.

The irradiation apparatus may be any of a handheld apparatus and a stationary apparatus. The irradiation apparatus may comprise a handheld applicator. The applicator may house some or all of the components of the irradiation apparatus.

The irradiation apparatus may further comprise a base unit. The base unit may comprise a portable box with a transport handle. The base unit may comprise a power supply interface for connection with an external power supply and/ or a power supply unit, e.g. comprising a battery or accumulator. The base unit may further comprise a display and/ or a control unit for the irradiation apparatus. A communication link may ("freely") extend between, on the one hand, the base unit and, on the other hand, the handheld applicator. The communication link may be adapted for any of data transmission, for controlling the irradiation apparatus, and/ or power supply of the irradiation device. The communication link may also be adapted with an operation channel to supply substances and/ or to collect samples or probes.

The UV germicidal irradiation apparatus may further comprise an additional radiation source being adapted to generate additional radiation. The additional radiation source is adapted to generate radiation in any range of the wavelength range of 300 - 320 nm and 360 - 700 nm.

The additional radiation source may be radiation-wisely coupled to the radiation output guide via at least one of the filter arrangement and the radiation interface, so that additional radiation from the additional radiation source may propagate to the radiation output guide. Alternatively, the additional radiation source may be radiation-wisely coupled to the radiation output guide via at least one of an additional filter arrangement and an additional radiation interface, so that additional radiation from the additional radiation source may propagate to the radiation output guide. Alternatively, the additional radiation source may be radiation-wisely coupled to an additional radiation output guide of the UV germicidal irradiation apparatus via at least one of an additional filter arrangement and an additional radiation interface, so that additional radiation from the additional radiation source may propagate to additional radiation output guide.

The additional filter arrangement may comprise at least one additional optical filter for optically filtering-out at least one wavelength in at least one predefined wavelength range. The additional filter arrangement may be adapted to remove any carcinogenic radiation while transmitting germicidal radiation of the radiation source. The additional filter arrangement may be adapted to remove radiation in a wavelength range between 240 nm and 280 nm from radiation received from the UV germicidal radiation source. Alternatively, or in combination therewith, the additional filter arrangement may be adapted to remove radiation of at least one wavelength band in the wavelength range between 240 nm and 280 nm. The additional filter arrangement may be in particular adapted to remove radiation of a wavelength of 254 nm.

The additional filter arrangement may comprise at least one additional collimator adapted to output, in a radiation downstream direction, collimated radiation.

The at least one additional collimator may be adapted to output, in the radiation downstream direction, collimated radiation essentially in the longitudinal direction. The additional collimator may remove radiation from the radiation source that exceeds a certain angle with respect to the main radiation direction.

The additional filter arrangement may comprise at least one additional optical filter radiationally upstream the at least one additional collimator and at least one additional optical filter radiationally downstream the at least one additional collimator. The additional filter arrangement may further comprise at least one additional collimator radiationally upstream the at least one additional optical filter and at least one collimator radiationally downstream the at least one additional optical filter.

The additional collimator may comprise at least one of non-reflective plastic or ceramic. The additional collimator may be formed as at least one of a honey-combed structure, a circularly perforated structure, a square-shape perforated structure, and a grid-like structure.

The additional radiation output guide may comprise at least one the following: a fused silica glass, silica, transparent UV-C material, a metal-coated (hollow) waveguide, waveguide having total-reflection properties, a UV-C transparent sapphire, a UV-C transparent diamond, and a UV-C transparent semi-conductor material.

The additional radiation output guide may be formed as a solid rod, an optical waveguide, an optical fiber, a tube, a sphere, a hemisphere, a part of a sphere, and a cone.

The additional radiation source may comprise at least one of the following: a UV or visible light lamp, a UV or visible light laser, a UV or visible light LED, an excimer laser, an excimer lamp, a Xe-Cl excimer lamp, or a Xe-Cl excimer laser.

The additional radiation output guide may comprise at least one additional flexible segment.

The apparatus may comprise an additional actuating device being operatively coupled to the additional flexible segment of the additional radiation output guide and adapted to, upon actuation, move the additional flexible segment of the additional radiation output guide.

The UV germicidal irradiation apparatus may be used for destroying at least one of the following: germs, bacteria, multi-resistant bacteria, Staphylococcus aureus, fungi, virus, influenza virus, human papillomaviruses, Corona-Viruses, Candida albans, Mycobacteria, Spores of bacteria, SARS, MERS, Covid, SARS-CoV, MERS-CoV, SARS-CoV-2, COVID-19, pathogens causing skin diseases, pathogens causing mucosa diseases.

The UV germicidal irradiation apparatus may be used for outputting radiation from the radiation applicator onto at least one of the following:
- tissue in a body orifice,
- tissue in a mouth of a living body,
- tissue in a pharynx area,
- tissue in nasal area,
- tissue in genital area,
- tissue of the vaginal canal,
- tissue of the cervix,
- tissue of the anal canal,
- tissue of an open wound,
- tissue of skin,
- a surface of an object,
- onto tissue in fluid,
- onto fluid
- onto a gas volume
- onto an air volume.

The invention is defined in the claims. Other embodiments, examples, modifications, uses or methods are not a part of the invention and are disclosed for illustrative purposes only.

### BRIEF DESCRIPTION OF THE DRAWINGS

Features of examples will be described, by way of example, in the following detailed description with reference to the accompanying drawings in, which like reference numerals correspond to similar, though perhaps not identical, components. For the sake of brevity, reference numerals or features having a previously described function may or may not be described in connection with other drawings in, which they appear.

Non-limiting examples will now be described with reference to the accompanying drawings, in, which:
Fig. 1 shows a schematic illustration of an exemplary UV germicidal irradiation apparatus.
Fig. 2 - 5 show schematic illustrations of exemplary filter arrangements of an UV germicidal irradiation apparatus.
Fig. 6 shows a schematic illustration of an exemplary collimator of a UV germicidal irradiation apparatus.
Fig. 7 shows a schematic illustration of an exemplary UV germicidal irradiation apparatus.
Fig. 8 shows a further schematic illustration of the exemplary UV germicidal irradiation apparatus of Fig. 7.
Fig. 9 - 11 shows schematic illustrations of an exemplary applicators of an UV germicidal irradiation apparatus.
Fig. 12 shows a schematic illustration of a use example of the exemplary applicator of Fig. 11.
Fig. 13 and Fig. 14 show schematic illustrations of further exemplary UV germicidal irradiation apparatuses.

### DETAILED DESCRITPION

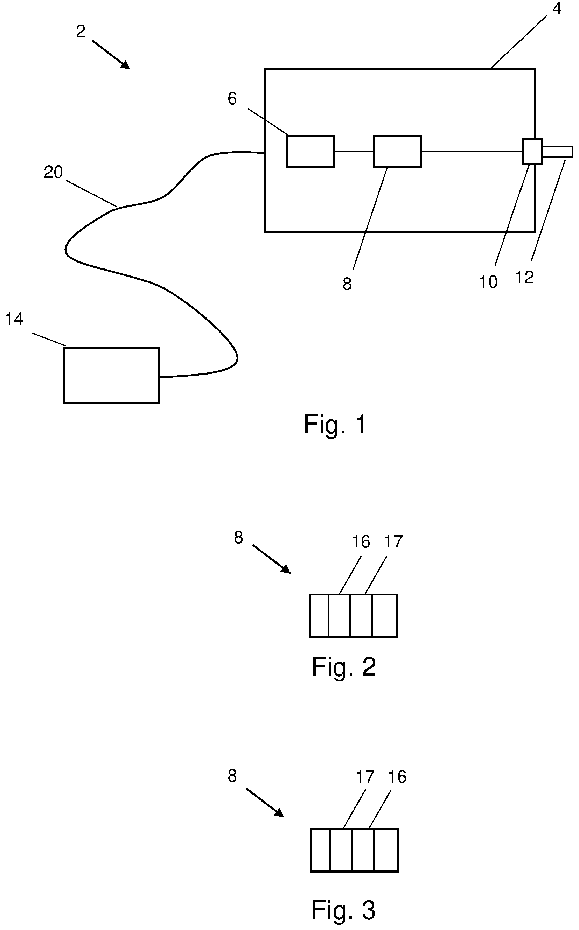

Fig. 1 shows a simplified, schematic illustration of a UV germicidal irradiation apparatus 2 according to present invention. The irradiation apparatus 2 comprises a housing 4 accommodating a UV germicidal radiation source 6, a filter arrangement 8 and a radiation interface 10. The radiation interface 10 can be considered as connection and output part that both enables structurally connecting a radiation output guide 12 to the housing 4 and radiation-wisely coupling the radiation output guide 12 to radiation originating at the radiation source 6 so that radiation may be outputted to the radiation output guide 12. The radiation output guide 12 and the radiation interface 10 are non-releasably or releasably connected such that different types of radiation output guides can be connected to the radiation interface 10. The functional unit provided by the housing 4 can be also referred to as applicator.

The radiation source 6 is preferably adapted to generate UV germicidal radiation in a wavelength between 190nm and 230 nm. Preferably, the radiation source 6 generates single-line or narrow band wavelength in the range of 222 nm. The radiation source 6 comprises a UV-C lamp, in particular a Kr-Cl-excimer lamp to generate the respective wavelength.

The filter arrangement 8 is adapted to remove any carcinogenic radiation, that is to say radiation and/ or a radiation band in a wavelength range between 240 nm and 280 nm. The filter arrangement 8 is arranged downstream the radiation source 6 and upstream the radiation interface 10. Thus, generated radiation passes through the filter arrangement 8 and the radiation interface 10 on its way from the radiation source 6 to the distal end of the output guide. The filter arrangement 8 may comprise a quartz glass plate with dielectric coating.

The radiation output guide 12 extends longitudinally from the radiation interface 10. The radiation output guide 12 is adapted to guide radiation via the radiation interface 10 towards target issue.

The apparatus further comprises a base unit and a communication link 20, which serves e.g. for power or data transmission between the base unit 14 and the functional elements arranged within the housing 4.

Fig. 2 - 5 show schematic illustrations of a filter arrangement 8, which comprises at least one collimator 17 and at least one optical filter 16. In Fig. 2, the filter arrangement is such that the optical filter 16 is arranged radially upstream the collimator 17. Fig. 3 shows a filter arrangement 8, in which the optical filter is arranged radially downstream the collimator 17.

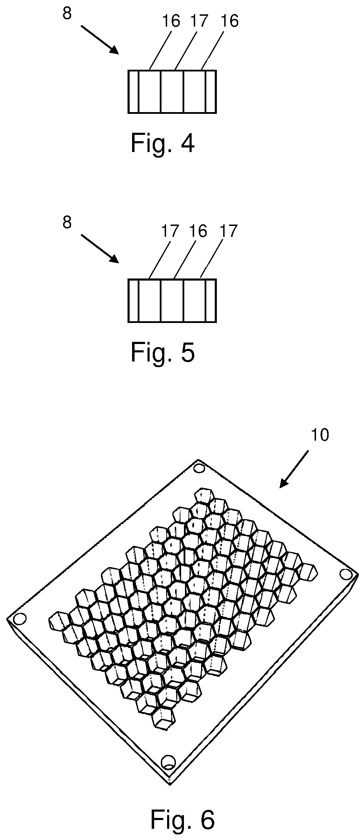

Fig. 4 shows a filter arrangement 8 comprising two optical filters 16 and a collimator 17. One optical filter 16 is arranged radiationally upstream the collimator 17 and one optical filter is arranged radiationally downstream the collimator.

Fig. 5 shows a filter arrangement comprising two collimators 17 and an optical filter 16. The optical filter 16 is radiationally arranged between the two collimators 17. Deviating filter arrangements, e.g. with an equal number of collimators 17 and optical filters 16, may be possible as well (not shown).

Fig. 6 shows a schematic illustration of a collimator 17 of the irradiation apparatus 2. The collimator 17 has a honey-combed structure and is adapted to produce parallel radiation from the radiation source.

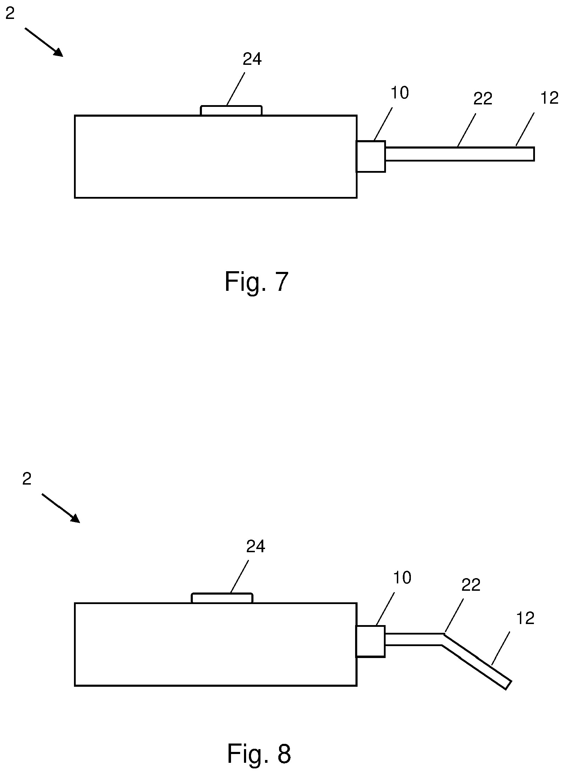

Fig. 7 shows a simplified handheld irradiation apparatus 2, which comprises an output guide 12 releasably connected to the radiation interface 10. The output guide comprises a flexible segment 22. The flexible segment 22 is shown in a straight position. The irradiation apparatus 2 also comprises an actuating device 24, which is coupled to the flexible segment 22 of the output guide 12.

Fig.8 shows an embodiment of the irradiation apparatus 2 of Fig. 7, in, which the actuating device 24 has been actuated such that the flexible segment of 22 of the output guide 12 has been actuated to be flexed. The flexible segment 22 of the output guide 12 remains in this state until further actuation of the actuating device 24. The actuating device 24 may be actuated during use of the irradiation apparatus 2, e.g. when the radiation output guide 12 is inside a body office of a patient. This way, the direction of irradiation may be adapted without needing to reintroduce the radiation output guide 12 into the patient.

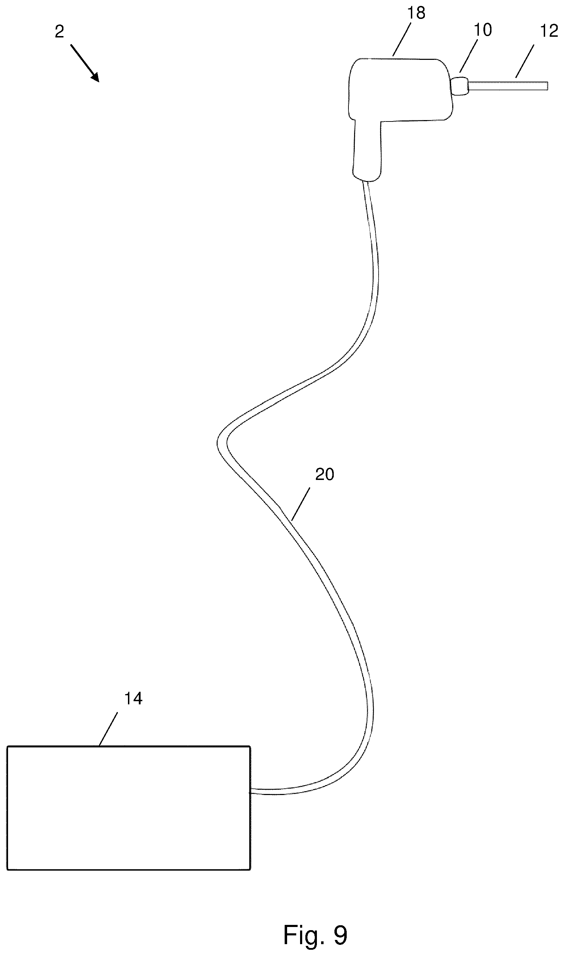

Fig. 9 illustrates a further embodiment of the irradiation apparatus 2, which comprises the base unit 14 and a handheld applicator 18. The applicator 18 may be ergonomically designed, e.g. as shown as pistol-grip like shape. The communication link 20 may serve to transmit data and/ or to supply electric power between the base unit 14 and the applicator 18. The communication link 20 may also serve comprise an operation channel to supply substances and/ or to collect samples or probes. Here, the radiation output guide 12 may comprise a solid quartz glass rod. The quartz rod is releasably coupled to the radiation interface 10. In some cases, the base unit may comprise a display and/ or a charging station for wire or wireless charging of the radiation applicator (not shown).

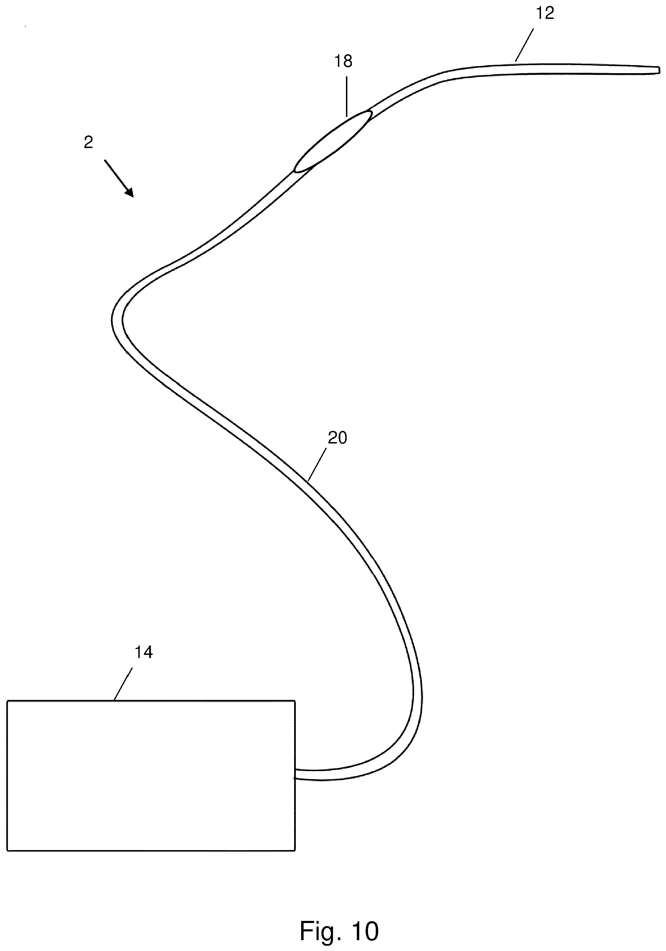

Fig. 10 shows another embodiment of the irradiation apparatus 2 comprising a base unit 14 and a radiation applicator 18, which has a shape that "aligns" with the communication link 20 and/ or and the radiation output guide 12. Such a radiation applicator may still even with a smaller form factor incorporate, e.g., control elements for controlling the output of radiation, an actuation device for the radiation output guide (or a flexible part thereof) etc. Such a radiation applicator may be used in situations with space having more limited freedom of movement.

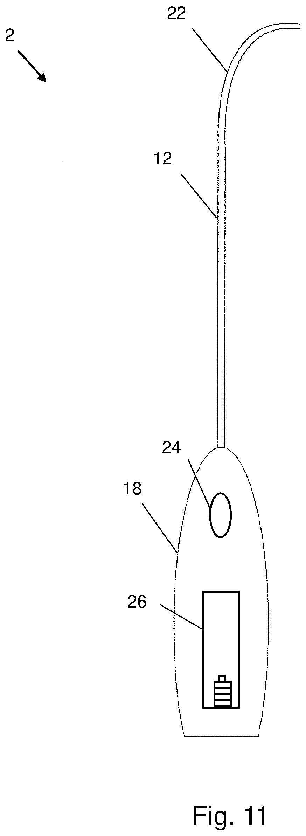

In Fig. 11, a further simplified example of the irradiation apparatus 2 is shown. In this example, the irradiation apparatus 2 is a handheld apparatus. The apparatus comprises an applicator 18, from which the radiation output guide 12 protrudes. The output guide 12 also comprises a flexible segment 22, which can be moved upon actuation of the actuation device 24. The handheld irradiation apparatus 2 further comprises a control unit 26, which allows the user to control the radiation source 6. The control unit 26 further comprises an indicator indicating the charging status of the battery. The handheld irradiation apparatus 2 also includes a charging interface for wire or wireless charging of the battery (not shown).

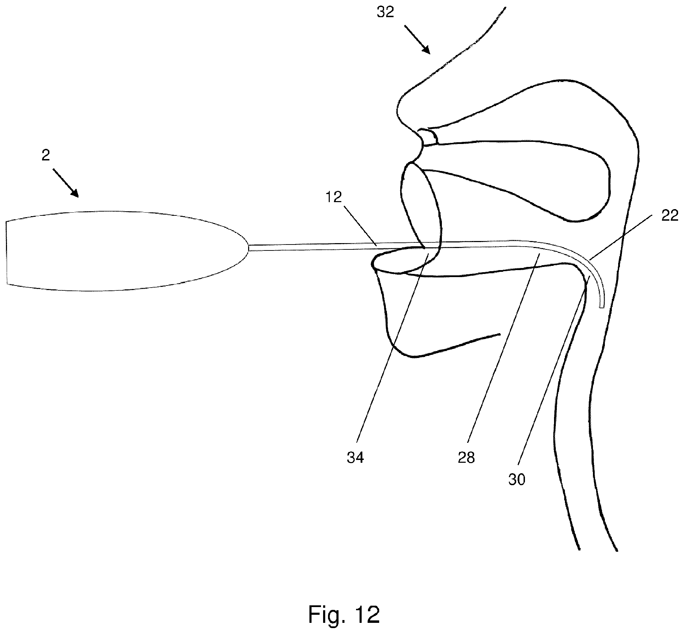

Fig. 12 shows an exemplary use of the example UV irradiation apparatus 2 discussed in connection with Fig. 11. Here, the target tissue to be irradiated is in the pharynx 28 area of a patient 32. For this purpose, the output guide 12 is introduced through the mouth 34 of the patient 32. The flexible segment 22 of the output guide 12 is flexed such that areas of the laryngopharynx 30 are irradiated. The flexible segment 22 of the output guide 12 may be adapted during use of the UV irradiation apparatus such that different areas of the pharynx can be irradiated without the need of reintroducing the output guide 12 of the irradiation apparatus 2 to the patient. The output guide 12 of the irradiation apparatus focuses the generated radiation such that it passes through body fluids or mucous membranes of the body cavity in order to reach the target tissue.

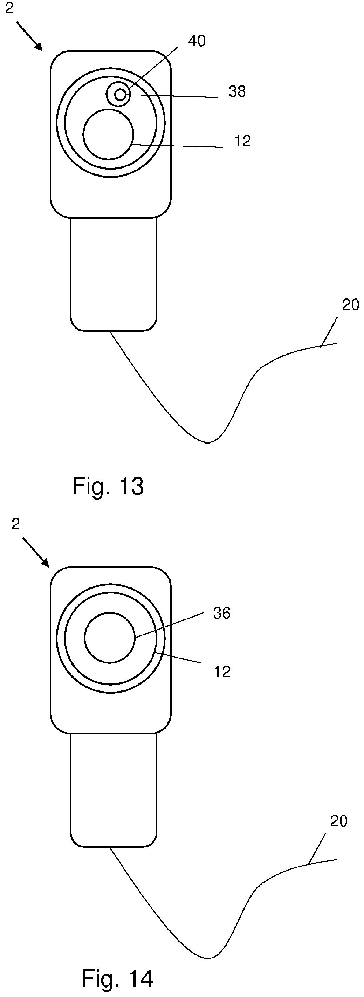

Fig. 13 and Fig. 14 illustrate additional elements that may be included to an irradiation apparatus 2. As described above, a germicidal UV irradiation apparatus may comprise any or a combination of: at least one additional radiation source, an additional radiation output guide, a tissue parameter determining device, an imaging device, a substance application device, and the like.

Fig. 13 shows an operational channel 40 through, which different additional elements, according to a use case, can be positioned on target tissue inside or outside the body. In this example, a substance application device 38 may be introduced through the operation channel 40 of the irradiation device 2 into a body cavity. Otherwise, the additional element may be permanently arranged to the irradiation apparatus (not shown). The one or more additional element may be arranged adjacent to the radiation output guide 12 of the irradiation apparatus 2. The substance application device 38 may be adapted to apply a substance that enhanced the effects of UV-C radiation. The substance application device 38 may be used while or independent from operating the radiation source. The substance application device 38 may be supplied with the respective substance via the communication link 20, which connects the substance application device to a base unit.

Fig. 14 illustrates another example of the irradiation apparatus comprising an additional element, which is arranged coaxially to the radiation output guide 12 of the irradiation apparatus 2. In this case, the additional element is a determining device 36, which determines the germ burden of target tissue. To do so, the determining device 36 protrudes a smear tissue, which, upon minimal movement, samples the respective germ burden of tissue. The smear tissue may then be withdrawn and analyzed.

The additional element and the output guide 12 may be in the same or different plane of the irradiation apparatus 2 in Fig. 13 and Fig. 14,

It is to be understood that features discussed in connection with one embodiment may be combined with features of other embodiments. By no means, the embodiments described herein limit the present invention.

| **List of reference signs** | |
|---|---|
| 2 | UV germicidal irradiation apparatus |
| 4 | housing |
| 6 | UV germicidal radiation source |
| 8 | filter arrangement |
| 10 | radiation interface |
| 12 | radiation output guide |
| 14 | base unit |
| 16 | optical filter |
| 17 | collimator |
| 18 | handheld UV germicidal radiation applicator |
| 20 | communication link |
| 22 | flexible segment |
| 24 | actuating device |
| 26 | control unit |
| 28 | pharynx |
| 30 | laryngopharynx |
| 32 | patient |
| 34 | patient's mouth |
| 36 | determining device |
| 38 | substance application device |
| 40 | operational channel |
| 42 | motion detector |

## Claims

1. An UV germicidal irradiation apparatus (2) for irradiation of target tissue of a living body, comprising:
- an UV germicidal radiation source (6),
- a filter arrangement (8),
- a radiation interface (10),
- a radiation output guide (12);
wherein
- the UV germicidal radiation source (6) is adapted to output UV germicidal radiation in a wavelength range between 190 nm and 230 nm;
- the UV germicidal radiation source (6) and the filter arrangement (8) are arranged to successively be traversed by radiation propagating in downstream direction to transmit radiation from the UV germicidal radiation source (6) to the filter arrangement (8),
- the filter arrangement (8) is adapted to remove radiation in a wavelength range between 240 nm and 280 nm from radiation received from the UV germicidal radiation source (6),
- the filter arrangement (8) and the radiation interface (10) being arranged to successively be traversed by radiation propagating in downstream direction to transmit radiation from the filter arrangement (8) to the radiation interface (10),
- the radiation interface (10) being adapted to output radiation received from the filter arrangement (8);
- the radiation interface (10) and the radiation output guide (12) being connected to each other and arranged to successively be traversed by radiation propagating in downstream direction to transmit radiation from the radiation interface (10) to the radiation output guide (12),
wherein
- the radiation output guide (12) extends, from the radiation interface (10), longitudinally in a downstream direction wherein its diameter is smaller than its length and is adapted to guide radiation towards target tissue;
wherein the radiation output guide (12) comprises at least on flexible segment (22) and an actuating device (24), which is operatively coupled to the at least one flexible segment (22) of the radiation output guide (12).

2. An UV germicidal irradiation apparatus (2) according to claim 1, wherein the radiation output guide (12) is fully flexible and wherein the flexible segment (22) can be moved in a controlled manner.

3. An UV germicidal irradiation apparatus (2) according to claim 1, wherein the radiation source (6) is adapted to output at least one of
- at least one wavelength in the wavelength range between 190 nm and 230 nm,
- at least one wavelength band in the wavelength range between 190 nm and 230 nm,
- at least one radiation of a wavelength in any of the range of 222 nm and the range of 207 nm.

4. An UV germicidal irradiation apparatus (2) according to one of the preceding claims, wherein the filter arrangement (8) is adapted to remove at least one of
- radiation of at least one wavelength in the wavelength range between 240 nm and 280 nm;
- radiation of at least one wavelength band in the wavelength range between 240 nm and 280 nm.

5. A UV germicidal irradiation apparatus (2) according to one of the preceding claims, wherein the filter arrangement (8) comprises at least one optical filter for optically filtering- out at least one wavelength in at least one predefined wavelength range.

6. A UV germicidal irradiation apparatus (2) according to one of the preceding claims, wherein the filter arrangement (8) comprises at least one collimator adapted to output, in a radiation downstream direction, collimated radiation.

7. A UV germicidal irradiation apparatus (2) according to the preceding claim, wherein the at least one collimator is adapted to output, in the radiation downstream direction, collimated radiation essentially in the longitudinal direction.

8. A UV germicidal irradiation apparatus (2) according to claim 4 and one of the claims 5 and 6, wherein the filter arrangement (8) comprises
- at least one optical filter radiationally upstream the at least one collimator and at least one optical filter radiationally downstream the at least one collimator,
- at least one collimator radiationally upstream the at least one optical filter and at least one collimator radiationally downstream the at least one optical filter.

9. A UV germicidal irradiation apparatus (2) according to one of the claims 4 to 7, wherein the collimator comprises at least one of non-reflective plastic or ceramic.

10. A UV germicidal irradiation apparatus (2) according to one of the claims 4 to 8, wherein the collimator is formed as at least one of
- a honey-combed structure,
- a circularly perforated structure,
- a square-shape perforated structure,
- a grid-like structure.

11. A UV germicidal irradiation apparatus (2) according to one of the preceding claims, wherein the radiation output guide (12) comprises at least one of
- fused silica glass,
- silica,
- transparent UV-C material,
- a metal-coated (hollow) waveguide,
- waveguide having total-reflection properties,
- UV-C transparent sapphire,
- UV-C transparent diamond,
- UV-C transparent semi-conductor material.

12. A UV germicidal irradiation apparatus (2) according to one of the preceding claims, wherein the radiation output guide (12) is formed as at least one of a
- solid rod,
- optical waveguide,
- optical fiber,
- tube,
- sphere,
- hemisphere,
- part of a sphere,
- cone.

13. A UV germicidal irradiation apparatus (2) according to one of the preceding claims, wherein the radiation source (6) comprises at least one of
- a UV-C lamp,
- a UV-C laser,
- a UV-C LED,
- an excimer laser,
- an excimer lamp,
- a Kr-Br excimer lamp,
- a Kr-Br excimer laser,
- a Kr-Cl excimer lamp,
- a Kr-Cl excimer laser.

14. The UV germicidal irradiation apparatus (2) according to one of the preceding claims, further comprising at least one of
- a tissue parameter determining device being adapted to a temperature of tissue,
- a tissue parameter determining device being adapted to a color of tissue,
- a tissue parameter determining device being adapted to a melanin content of tissue,
- a tissue parameter determining device being adapted to a thickness of tissue,
- a tissue parameter determining device being adapted to a pathogen burden of tissue,
- a tissue parameter determining device being adapted to a germ burden of tissue,
- an imaging device adapted to generate images of a surface of tissue,
- an imaging device adapted to generate images of internal structures of tissue,
- an imaging device including a video camera,
- an imaging device including a microscope,
- an imaging device using optical coherence tomography,
- an imaging device using ultrasound,
- n photodynamic imaging device,
- an imaging device adapted to visualize a pathogen burden of tissue,
- an imaging device adapted to visualize a germ burden of tissue.

## Patentansprüche

1. UV-Entkeimungsbestrahlungs-Vorrichtung (2) zur Bestrahlung von Zielgewebe eines Lebendkörpers, umfassend:
- eine UV-Entkeimungsstrahlungs-Quelle (6),
- eine Filteranordnung (8),
- eine Strahlungsschnittstelle (10),
- einen Strahlungsausgangsleiter (12);
wobei
- die UV-Entkeimungsstrahlungs-Quelle (6) ausgelegt ist, um UV-Entkeimungsstrahlung in einem Wellenlängenbereich zwischen 190 nm und 230 nm auszugeben;
- die UV-Entkeimungsstrahlungs-Quelle (6) und die Filteranordnung (8) angeordnet sind, um sukzessiv von Strahlung, die in Stromabwärtsrichtung sich ausbreitet, durchlaufen zu werden, um Strahlung von der UV-Entkeimungsstrahlungs-Quelle (6) zur Filteranordnung (8) zu übertragen,
- die Filteranordnung (8) ausgelegt ist, um Strahlung in einem Wellenlängenbereich zwischen 240 nm und 280 nm aus der von der UV-Entkeimungsstrahlungs-Quelle (6) empfangenen Strahlung zu entfernen,
- die Filteranordnung (8) und die Strahlungsschnittstelle (10) angeordnet sind, um sukzessiv von Strahlung durchlaufen zu werden, die sich in Stromabwärtsrichtung ausbreitet, um Strahlung von der Filteranordnung (8) zur Strahlungsschnittstelle (10) zu übertragen,
- die Strahlungsschnittstelle (10) ausgelegt ist, um von der Filteranordnung (8) empfangene Strahlung auszugeben;
- die Strahlungsschnittstelle (10) und der Strahlungsausgangsleiter (12) miteinander verbunden und angeordnet sind, um sukzessiv von Strahlung durchlaufen zu werden, die sich in Stromabwärtsrichtung ausbreitet, um Strahlung von der Strahlungsschnittstelle (10) zum Strahlungsausgangsleiter (12) zu übertragen,
wobei
- der Strahlungsausgangsleiter (12) von der Strahlungsschnittstelle (10) aus in Stromabwärtsrichtung länglich erstreckt, wobei sein Durchmesser kleiner als seine Länge ist, und dazu ausgelegt ist, um Strahlung zum Zielgewebe zu leiten;
wobei der Strahlungsausgangsleiter (12) mindestens ein flexibles Segment (22) und eine Betätigungsvorrichtung (24) umfasst, die funktionsfähig mit dem mindestens einen flexiblen Segment (22) des Strahlungsausgangsleiters (12) gekoppelt ist.

2. UV-Entkeimungsbestrahlungs-Vorrichtung (2) nach Anspruch 1, wobei der Strahlungsausgangsleiter (12) vollständig flexibel ist und wobei das flexible Segment (22) auf kontrollierte Weise bewegt werden kann.

3. UV-Entkeimungsbestrahlungs-Vorrichtung (2) nach Anspruch 1, wobei die Strahlungsquelle (6) ausgelegt ist, um mindestens eines auszugeben:
- mindestens eine Wellenlänge im Wellenlängenbereich zwischen 190 nm und 230 nm,
- mindestens ein Wellenlängenband im Wellenlängenbereich zwischen 190 nm und 230 nm,
- mindestens eine Strahlung einer Wellenlänge in irgendeinem des Bereichs von 222 nm und des Bereichs von 207 nm.

4. UV-Entkeimungsbestrahlungs-Vorrichtung (2) nach einem der vorstehenden Ansprüche, wobei die Filteranordnung (8) ausgelegt ist, um mindestens eines zu entfernen:
- eine Strahlung mindestens einer Wellenlänge im Wellenlängenbereich zwischen 240 nm und 280 nm;
- eine Strahlung mindestens eines Wellenlängenbandes im Wellenlängenbereich zwischen 240 nm und 280 nm.

5. UV-Entkeimungsbestrahlungs-Vorrichtung (2) nach einem der vorstehenden Ansprüche, wobei die Filteranordnung (8) mindestens einen optischen Filter zum optischen Herausfiltern mindestens einer Wellenlänge in mindestens einem vordefinierten Wellenlängenbereich umfasst.

6. UV-Entkeimungsbestrahlungs-Vorrichtung (2) nach einem der vorstehenden Ansprüche, wobei die Filteranordnung (8) mindestens einen Kollimator umfasst, der ausgelegt ist, um in einer Strahlungsabwärtsrichtung kollimierte Strahlung auszugeben.

7. UV-Entkeimungsbestrahlungs-Vorrichtung (2) nach einem der vorstehenden Ansprüche, wobei der mindestens eine Kollimator ausgelegt ist, um in der Strahlungsabwärtsrichtung im Wesentlichen in Längsrichtung kollimierte Strahlung auszugeben.

8. UV-Entkeimungsbestrahlungs-Vorrichtung (2) nach Anspruch 4 und einem der Ansprüche 5 und 6, wobei die Filteranordnung (8) umfasst:
- mindestens einen optischen Filter strahlungsmäßig stromaufwärts des mindestens einen Kollimators und mindestens einen optischen Filter strahlungsmäßig stromabwärts des mindestens einen Kollimators,
- mindestens einen Kollimator strahlungsmäßig stromaufwärts des mindestens einen optischen Filters und mindestens einen Kollimator strahlungsmäßig stromabwärts des mindestens einen optischen Filters.

9. UV-Entkeimungsbestrahlungs-Vorrichtung (2) nach einem der Ansprüche 4 bis 7, wobei der Kollimator mindestens eines von nicht reflektierendem Kunststoff oder Keramik umfasst.

10. UV-Entkeimungsbestrahlungs-Vorrichtung (2) nach einem der Ansprüche 4 bis 8, wobei der Kollimator als mindestens eines von geformt ist:
- eine Wabenstruktur,
- eine kreisförmig perforierte Struktur,
- eine quadratisch perforierte Struktur,
- eine gitterartige Struktur.

11. UV-Entkeimungsbestrahlungs-Vorrichtung (2) nach einem der vorstehenden Ansprüche, wobei der Strahlungsausgangsleiter (12) mindestens eines umfasst:
- Quarzglas,
- Siliziumdioxid,
- transparentes UV-C-Material,
- metallbeschichteter (hohler) Wellenleiter,
- Wellenleiter aufweisend Totalreflexionseigenschaften,
- UV-C-transparenten Saphir,
- UV-C-transparenten Diamant,
- UV-C-transparentes Halbleitermaterial.

12. UV-Entkeimungsbestrahlungs-Vorrichtung (2) nach einem der vorstehenden Ansprüche, wobei der Strahlungsausgangsleiter (12) als mindestens eines geformt ist:
- massiver Stab,
- optischer Wellenleiter,
- optische Faser,
- Röhre,
- Kugel,
- Halbkugel,
- Teil einer Kugel,
- Kegel.

13. UV-Entkeimungsbestrahlungs-Vorrichtung (2) nach einem der vorstehenden Ansprüche, wobei die Strahlungsquelle (6) mindestens eines umfasst:
- eine UV-C-Lampe,
- einen UV-C-Laser,
- eine UV-C-LED,
- einen Excimerlaser,
- eine Excimerlampe,
- eine Kr-Br-Excimerlampe,
- einen Kr-Br-Excimer,
- eine Kr-Cl-Excimerlampe,
- einen Kr-Cl-Excimerlaser.

14. UV-Entkeimungsbestrahlungs-Vorrichtung (2) nach einem der vorstehenden Ansprüche, die ferner mindestens eines umfasst:
- eine Gewebeparameterbestimmungs-Vorrichtung, die auf eine Temperatur eines Gewebes ausgelegt ist,
- eine Gewebeparameterbestimmungs-Vorrichtung, die auf eine Farbe eines Gewebes ausgelegt ist,
- eine Gewebeparameterbestimmungs-Vorrichtung, die an ein Melaningehalt eines Gewebes angepasst ist,
- eine Gewebeparameterbestimmungs-Vorrichtung, die an eine Dicke eines Gewebes angepasst ist,
- eine Gewebeparameterbestimmungs-Vorrichtung, die an eine Pathogenbelastung eines Gewebes angepasst ist,
- eine Gewebeparameterbestimmungs-Vorrichtung, die an eine Keimbelastung eines Gewebes angepasst ist,
- eine Bildgebungsvorrichtung, die zur Erzeugung von Bildern einer Gewebeoberfläche ausgelegt ist,
- eine Bildgebungsvorrichtung, die zur Erzeugung von Bildern innerer Strukturen des Gewebes ausgelegt ist,
- eine Bildgebungsvorrichtung, die eine Videokamera beinhaltet,
- eine Bildgebungsvorrichtung, die ein Mikroskop beinhaltet,
- eine Bildgebungsvorrichtung, die optische Kohärenztomographie verwendet,
- eine Bildgebungsvorrichtung, die Ultraschall verwendet,
- eine Photodynamischebildgebungs-Vorrichtung,
- eine Bildgebungsvorrichtung, die zur Visualisierung einer Pathogenbelastung von Gewebe ausgelegt ist,
- eine Bildgebungsvorrichtung, die zur Visualisierung einer Keimbelastung von Gewebe ausgelegt ist.

## Revendications

1. Appareil d'irradiation UV germicide (2) pour l'irradiation de tissu cible d'un corps vivant, comprenant :
- une source de rayonnement UV germicide (6),
- un agencement de filtre (8),
- une interface de rayonnement (10),
- un guide de sortie de rayonnement (12) ;
dans lequel
- la source de rayonnement UV germicide (6) est conçue pour émettre un rayonnement UV germicide dans une plage de longueurs d'onde entre 190 nm et 230 nm ;
- la source de rayonnement UV germicide (6) et l'agencement de filtre (8) sont agencés pour être traversés successivement par un rayonnement se propageant dans la direction aval afin de transmettre le rayonnement de la source de rayonnement UV germicide (6) à l'agencement de filtre (8),
- l'agencement de filtre (8) est conçu pour éliminer le rayonnement dans une plage de longueurs d'onde entre 240 nm et 280 nm du rayonnement reçu de la source de rayonnement UV germicide (6),
- l'agencement de filtre (8) et l'interface de rayonnement (10) étant agencés pour être traversés successivement par un rayonnement se propageant dans la direction aval afin de transmettre le rayonnement de l'agencement de filtre (8) à l'interface de rayonnement (10),
- l'interface de rayonnement (10) étant conçue pour émettre le rayonnement reçu de l'agencement de filtre (8) ;
- l'interface de rayonnement (10) et le guide de sortie de rayonnement (12) étant connectés l'un à l'autre et agencés pour être traversés successivement par un rayonnement se propageant dans la direction aval afin de transmettre le rayonnement de l'interface de rayonnement (10) au guide de sortie de rayonnement (12),
dans lequel
- le guide de sortie de rayonnement (12) s'étend, à partir de l'interface de rayonnement (10), longitudinalement dans une direction aval son diamètre étant inférieur à sa longueur et étant conçu pour guider le rayonnement vers le tissu cible ;
dans lequel le guide de sortie de rayonnement (12) comprend au moins un segment flexible (22) et un dispositif d'actionnement (24), qui est couplé fonctionnellement audit au moins un segment flexible (22) du guide de sortie de rayonnement (12).

2. Appareil d'irradiation UV germicide (2) selon la revendication 1, dans lequel le guide de sortie de rayonnement (12) est entièrement flexible et dans lequel le segment flexible (22) peut être déplacé de manière contrôlée.

3. Appareil d'irradiation UV germicide (2) selon la revendication 1, dans lequel la source de rayonnement (6) est conçue pour émettre au moins l'un de
- au moins une longueur d'onde dans la plage de longueurs d'onde entre 190 nm et 230 nm,
- au moins une bande de longueur d'onde dans la plage de longueurs d'onde entre 190 nm et 230 nm,
- au moins un rayonnement d'une longueur d'onde dans n'importe laquelle de la plage de 222 nm et la plage de 207 nm.

4. Appareil d'irradiation UV germicide (2) selon l'une des revendications précédentes, dans lequel l'agencement de filtre (8) est conçu pour éliminer au moins l'un de
- un rayonnement d'au moins une longueur d'onde dans la plage de longueurs d'onde entre 240 nm et 280 nm ;
- un rayonnement d'au moins une bande de longueur d'onde dans la plage de longueurs d'onde entre 240 nm et 280 nm.

5. Appareil d'irradiation UV germicide (2) selon l'une des revendications précédentes, dans lequel l'agencement de filtre (8) comprend au moins un filtre optique pour filtrer optiquement au moins une longueur d'onde dans au moins une plage de longueurs d'onde prédéfinie.

6. Appareil d'irradiation UV germicide (2) selon l'une des revendications précédentes, dans lequel l'agencement de filtre (8) comprend au moins un collimateur conçu pour émettre, dans une direction aval de rayonnement, un rayonnement collimaté.

7. Appareil d'irradiation UV germicide (2) selon la revendication précédente, dans lequel l'au moins un collimateur est conçu pour émettre, dans la direction aval du rayonnement, un rayonnement collimaté essentiellement dans la direction longitudinale.

8. Appareil d'irradiation UV germicide (2) selon la revendication 4 et l'une des revendications 5 et 6, dans lequel l'agencement de filtre (8) comprend
- au moins un filtre optique en amont en termes de rayonnement de l'au moins un collimateur et au moins un filtre optique en aval en termes de rayonnement de l'au moins un collimateur,
- au moins un collimateur en amont en termes de rayonnement dudit au moins un filtre optique et au moins un collimateur en aval en termes de rayonnement dudit au moins un filtre optique.

9. Appareil d'irradiation UV germicide (2) selon l'une des revendications 4 à 7, dans lequel le collimateur comprend au moins une matière plastique ou céramique non réfléchissante.

10. Appareil d'irradiation UV germicide (2) selon l'une des revendications 4 à 8, dans lequel le collimateur est formé comme au moins l'une de
- une structure alvéolée,
- une structure à perforations circulaires,
- une structure à perforations de forme carrée,
- une structure de type grille.

11. Appareil d'irradiation UV germicide (2) selon l'une des revendications précédentes, dans lequel le guide de sortie de rayonnement (12) comprend au moins l'un de
- un verre de silice fondu,
- la silice,
- un matériau transparent aux UV-C,
- un guide d'ondes (creux) revêtu de métal,
- un guide d'onde ayant des propriétés de réflexion totale,
- du saphir transparent aux UV-C,
- du diamant transparent aux UV-C,
- un matériau semi-conducteur transparent aux UV-C.

12. Appareil d'irradiation UV germicide (2) selon l'une des revendications précédentes,
dans lequel le guide de sortie de rayonnement (12) est formé comme au moins l'un de
- une tige pleine,
- un guide d'ondes optique,
- une fibre optique,
- un tube,
- une sphère,
- une hémisphère,
- une partie de sphère,
- un cone.

13. Appareil d'irradiation UV germicide (2) selon l'une des revendications précédentes, dans lequel la source de rayonnement (6) comprend au moins l'un de
- une lampe à UV-C,
- un laser UV-C,
- une DEL à UV,
- un laser excimère,
- une lampe excimère,
- une lampe excimère Kr-Br,
- un laser excimer Kr-Br,
- une lampe excimère Kr-CI,
- un laser excimère Kr-CI.

14. Appareil d'irradiation UV germicide (2) selon l'une des revendications précédentes, comprenant en outre au moins l'un de
- un dispositif de détermination de paramètres tissulaires étant conçu pour une température de tissu,
- un dispositif de détermination de paramètres tissulaires étant conçu pour une couleur de tissu,
- un dispositif de détermination de paramètres tissulaires étant conçu pour une teneur en mélanine de tissu,
- un dispositif de détermination de paramètres tissulaires étant conçu pour une épaisseur de tissu,
- un dispositif de détermination de paramètres tissulaires étant conçu pour une charge pathogène de tissu,
- un dispositif de détermination de paramètres tissulaires conçu pour une charge en germes de tissu,
- un dispositif d'imagerie conçu pour générer des images d'une surface de tissu,
- un dispositif d'imagerie conçu pour générer des images de structures internes de tissu,
- un dispositif d'imagerie incluant une caméra vidéo,
- un dispositif d'imagerie incluant un microscope,
- un dispositif d'imagerie utilisant la tomographie par cohérence optique,
- un dispositif d'imagerie par ultrasons,
- un dispositif d'imagerie photodynamique,
- un dispositif d'imagerie conçu pour visualiser une charge en pathogène de tissu,
- un dispositif d'imagerie conçu pour visualiser une charge en germes de tissu.
